(19) Europäisches Patentamt
European Patent Office
Office européen des brevets

(11) Veröffentlichungsnummer: **0 189 898**
**A1**

# (12) EUROPÄISCHE PATENTANMELDUNG

(21) Anmeldenummer: 86101103.9

(22) Anmeldetag: 28.01.86

(51) Int. Cl.⁴: C 07 D 471/04
A 61 K 31/44
//C07D471:04, C07D221:00

(30) Priorität: 29.01.85 DE 3502831

(43) Veröffentlichungstag der Anmeldung:
06.08.86 Patentblatt 86/32

(84) Benannte Vertragsstaaten:
AT CH DE FR GB IT LI

(71) Anmelder: **GÖDECKE AKTIENGESELLSCHAFT**
**Salzufer 16**
**D-1000 Berlin 10(DE)**

(72) Erfinder: **Satzinger, Gerhard, Dr.**
**Im Mattenbühl 7**
**D-7809 Denzlingen(DE)**

(72) Erfinder: **Hartenstein, Johannes, Dr.**
**Fohrenbühl 23**
**D-7801 Stegen-Wittental(DE)**

(72) Erfinder: **Mannhardt, Karl, Dr.**
**Pfauenstrasse 14**
**D-7807 Elzach-Oberprechtal(DE)**

(72) Erfinder: **Kleinschroth, Jürgen, Dr.**
**Freiburger Strasse 13**
**D-7809 Denzlingen(DE)**

(72) Erfinder: **Herrmann, Manfred, Dr.**
**Wolfweg 25**
**D-7811 St. Peter(DE)**

(72) Erfinder: **Fritschi, Edgar, Dr.**
**Am Scheuerwald 2**
**D-7811 St. Peter(DE)**

(72) Erfinder: **Tauschel, Horst-Dietmar, Dr.**
**Hinterdorfstrasse 29**
**D-7637 Ettenheim(DE)**

(72) Erfinder: **Wagner, Bernd, Dr.**
**Am Lossele 5**
**D-7809 Denzlingen(DE)**

(72) Erfinder: **Wolf, Günter, Dr.**
**Wildtalstrasse 40**
**D-7800 Freiburg(DE)**

(54) **1,6-Naphthyridinon-Derivate und Verfahren zu deren Herstellung.**

(57) Die Erfindung betrifft neue 1,6-Naphthyridinon-Derivate der allgemeinen Formel I

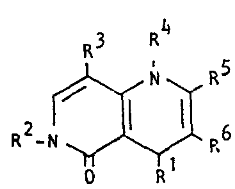

(I)

in welcher

$R^1$ einen unsubstituierten oder substituierten aromatischen oder heteroaromatischen Ring,

$R^2$ Wasserstoff, eine geradkettige oder verzweigte Alkyl-, Alkoxyalkyl-, Arylalkyl oder eine substituierte oder unsubstituierte Aminoalkylgruppe mit bis zu 10 Kohlenstoffatomen,

$R^3$ Wasserstoff, eine geradkettige oder verzweigte Alkylgruppe oder einen Alkoxycarbonylrest mit bis zu 4 Kohlenstoffatomen,

$R^4$ Wasserstoff oder eine Morpholinoethylgruppe,

$R^5$ eine geradkettige oder verzweigte Alkylgruppe mit bis zu 4 Kohlenstoffatomen oder eine Aminogruppe und

$R^6$ eine Carboxylgruppe oder einen Alkoxycarbonylrest, der bis zu 12 Kohlenstoffatome und gegebenenfalls auch ein Sauerstoff-, Schwefel- oder Stickstoffatom enthält, bedeuten sowie gegebenenfalls deren pharmakologisch unbedenkliche Salze.

Weiterhin werden Verfahren zur Herstellung derselben und deren Verwendung bei der Bekämpfung von Gefäßerkrankungen beschrieben.

Croydon Printing Company Ltd

BESCHREIBUNG

Die Erfindung betrifft neue 1,6-Naphthyridinon-Derivate der allgemeinen Formel I

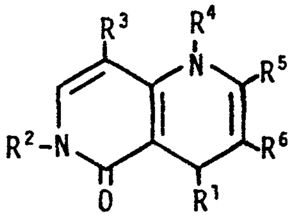

(I)

in welcher

$R^1$ einen unsubstituierten oder substituierten aromatischen oder heteroaromatischen Ring,

$R^2$ Wasserstoff, eine geradkettige oder verzweigte Alkyl-, Alkoxyalkyl-, Arylalkyl- oder eine substituierte oder unsubstituierte Aminoalkylgruppe mit bis zu 10 Kohlenstoffatomen,

$R^3$ Wasserstoff, eine geradkettige oder verzweigte Alkylgruppe oder einen Alkoxycarbonylrest mit bis zu 4 Kohlenstoffatomen,

$R^4$ Wasserstoff oder eine Morpholinoethylgruppe,

$R^5$ eine geradkettige oder verzweigte Alkylgruppe mit bis zu 4 Kohlenstoffatomen oder eine Aminogruppe und

$R^6$ eine Carboxylgruppe oder einen Alkoxycarbonylrest, der bis zu 12 Kohlenstoffatome und gegebenenfalls auch ein Sauerstoff-, Schwefel- oder Stickstoffatom enthält, bedeuten

sowie gegebenenfalls deren pharmakologisch unbedenkliche Salze.

Bevorzugt werden 1,6-Naphthyridinon-Derivate der allgemeinen Formel I,
in welcher

$R^1$ einen unsubstituierten oder einen vorzugsweise in 2- oder 3-Position durch Halogen, Nitro, Methyl, Methoxy, Difluormethoxy, Trifluormethoxy, Dimethyl- oder Diethylamino, Methylthio, oder Trifluormethyl substituierten Phenylrest, einen vorzugsweise in 2,3- durch Methoxy oder Methylendioxy oder in 2,3- oder 2,6-Position durch Halogenatome, die gleich oder verschieden sein können, disubstituierten Phenylrest, einen unsubstituierten Pyridyl-, Thienyl- oder 2,1,3-Benzoxadiazolylrest,

$R^2$ Wasserstoff, einen Methyl-, Ethyl-, n-Propyl- oder Isopropylrest, eine Alkoxyalkylgruppe der allgemeinen Formel II

$$-(CH_2)_n-O-R^7 \quad \text{(II)}$$

in welcher $R^7$ eine niedere geradkettige oder verzweigte Alkylgruppe darstellt und n den Wert 2 oder 3 bedeutet, oder eine Aminoalkylgruppe der allgemeinen Formel III

$$-(CH_2)_n-N\begin{matrix} R^8 \\ R^9 \end{matrix} \quad \text{(III)}$$

in welcher $R^8$ und $R^9$ gleich oder verschieden sein können und eine geradkettige oder verzweigte niedere Alkylgruppe darstellen, oder gemeinsam eine niedere Alkylengruppe bilden und n gleich 2 oder 3 ist,
bedeutet.

$R^3$ Wasserstoff, eine Methyl-, Ethyl- oder Isopropylgruppe, oder einen Alkoxycarbonylrest der allgemeinen Formel IV

$$-CO_2R^{10} \quad \text{(IV)}$$

in welcher $R^{10}$ eine Methyl-, Ethyl-, Propyl- oder Isopropylgruppe bedeutet,

0189898

$R^4$ Wasserstoff oder eine Morpholinoethylgruppe,

$R^5$ einen Methyl- oder Ethylrest oder eine Aminogruppe,

$R^6$ eine Carboxylgruppe oder einen Alkoxycarbonylrest der allgemeinen Formel V

$$-CO_2R^{11} \quad (V)$$

in welcher $R^{11}$ entweder einen Methyl-, Ethyl-, n-Propyl-, Isopropyl-, n-Butyl-, Isobutyl-, sek.-Butyl- oder tert. Butylrest, eine Alkoxylalkyl- oder Alkylthioalkylgruppe der allgemeinen Formeln VI und VII

$$-(CH_2)_n-O-R^{12} \quad (VI) \qquad -(CH_2)_n-S-R^{12} \quad (VII)$$

oder eine Aminoalkylgruppe der allgemeinen Formel VIII

$$-(CH_2)_n-N\begin{matrix} R^{13} \\ R^{14} \end{matrix} \quad (VIII)$$

worin $R^{12}$ eine niedere geradkettige oder verzweigte Alkylgruppe darstellt und $R^{13}$ und $R^{14}$ gleich oder verschieden sein können und Wasserstoff, eine geradkettige oder verzweigte niedere Alkylgruppe oder eine Benzylgruppe darstellen, oder gemeinsam eine niedere Alkylengruppe bilden und n gleich 2 oder 3 ist,
bedeuten.

Besonders bevorzugt sind Verbindungen in denen entweder $R^2$ und $R^4$ Wasserstoff bedeuten und wenn $R^3$ ebenfalls Wasserstoff bedeutet und $R^5$ eine Methylgruppe, dann bedeutet $R^{11}$ eine Dimethylaminoethyl-, Benzylmethylaminoethyl- oder Methylthioethylgruppe und wenn $R^{11}$ eine Ethylgruppe bedeutet, dann bedeutet $R^3$ eine Methyl- oder Ethoxycarbonylgruppe und $R^5$ eine Methyl- oder Ethylgruppe, oder bei denen

$R^2$ Wasserstoff- oder eine Benzylgruppe, $R^3$ Wasserstoff, $R^4$ Wasserstoff- oder eine Morpholinoethylgruppe, $R^5$ und $R^{11}$ eine Methylgruppe bedeuten.

Die Erfindung betrifft weiterhin ein Verfahren zur Herstellung von 1,6-Naphthyridin-Derivaten der allgemeinen Formel I, dadurch gekennzeichnet, daß man entweder

a) ein Dihydropyridin der allgemeinen Formel IX

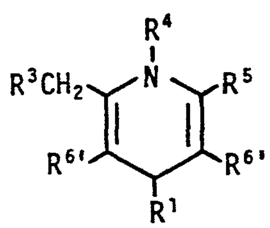

(IX)

in welcher $R^1$, $R^3$, $R^4$ und $R^5$ die oben genannte Bedeutung haben und $R^{6'}$ einen Alkoxycarbonylrest der allgemeinen Formel V darstellt, in Gegenwart einer Base mit s-Triazin umsetzt,

b) ein 1,4-Dihydropyridin der allgemeinen Formel IX mit einem Dialkylformamid-dialkylacetal der allgemeinen Formel X

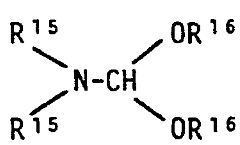

(X)

in welcher die Reste $R^{15}$ gleich oder verschieden sein können und eine Methyl- oder Ethylgruppe bedeuten und die Reste $R^{16}$ jeweils eine Alkylgruppe mit bis zu 4 Kohlenstoffatomen oder gemeinsam eine Alkylengruppe mit bis zu 3 Kohlenstoffatomen bedeuten, zur Reaktion bringt und die erhaltenen Verbindungen der allgemeinen Formel XI

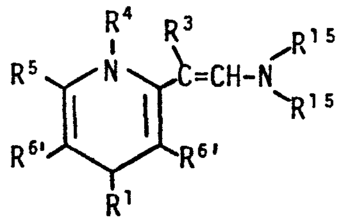 (XI)

in welcher $R^1$, $R^3$, $R^4$, $R^5$, $R^{6'}$ und $R^{15}$ die oben angegebene Bedeutung besitzen, mit Ammoniak umsetzt, oder

c) 2,4-Dihydroxypyridin mit einer Verbindung der allgemeinen Formel XII

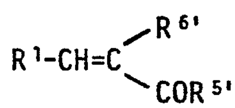 (XII)

in welcher $R^1$ und $R^{6'}$ die oben genannte Bedeutung haben und $R^{5'}$ eine geradkettige oder verzweigte Alkylgruppe mit bis zu 4 Kohlenstoffatomen bedeutet, in Gegenwart von Ammoniak umsetzt.

Die Verbindungen der allgemeinen Formel I mit $R^5$=$NH_2$ werden nach Verfahren a) aus den entsprechenden 2-Aminodihydropyridinen der allgemeinen Formel IX mit $R^5$=$NH_2$ hergestellt.

Die so erhaltenen Verbindungen der allgemeinen Formel XIII

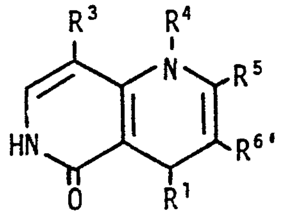

(XIII)

in welcher $R^1$, $R^3$, $R^4$, $R^5$ und $R^{6'}$ die obengenannte Bedeutung haben, werden gewünschtenfalls in an sich bekannter Weise mit einer Verbindung der allgemeinen Formel XIV

$$X\text{-}R^{2'} \quad (XIV)$$

in welcher $R^{2'}$ eine niedere geradkettige oder verzweigte Alkylgruppe, eine Alkoxyalkylgruppe der allgemeinen Formel II oder eine Aminoalkylgruppe der allgemeinen Formel III bedeutet, alkyliert, aminoalkyliert oder alkoxyalkyliert.

Verbindungen der allgemeinen Formel I, bei denen $R^6$ für eine Carboxylgruppe steht, werden hergestellt, indem man Verbindungen der allgemeinen Formel I, in denen $R^6$ für einen zur Esterspaltung geeigneten Alkoxycarbonylrest steht, in an sich bekannter Weise vorzugsweise in saurem Medium hydrolysiert.

Die Reaktion von s-Triazin mit den Verbindungen der allgemeinen Formel IX (Verfahren a) muß als überraschend angesehen werden, da aus der Literatur [Chem. Rev. 82, (1982)] hervorgeht, daß bei der Behandlung von 1,4-Dihydropyridinen mit starken Basen wie Natriumhydrid das Proton am Stickstoffatom unter Bildung eines Natriumamids entfernt wird, welches dann z.B. mit Alkylhalogeniden zu N-Alkylderivaten weiterreagiert. Im vorliegenden Fall findet die erwartete Reaktion nicht statt, sondern es erfolgt an der Methylgruppe eine Aminomethenylierung, die zu den Verbindungen der Formel XIII führt.

Die für das Verfahren a) und b) eingesetzten 1,4-Dihydropyridine der allgemeinen Formel IX sind bekannt [vgl. z.B. Chem. Rev. 82, (1982) S. 223] oder sie können in analoger Weise hergestellt werden.

Auch Verfahren c) ist chemisch eigenartig, da es als überraschend anzusehen ist, daß 2,4-Dihydroxypyridin in der beschriebenen Weise reagiert.

Die Verbindungen der allgemeinen Formel XII sind bekannt oder können nach literaturbekannten Verfahren hergestellt werden [Org. Reactions, Vol. 15 (1967) S. 204 ff.]. 2,4-Dihydroxypyridin ist Handelsprodukt.

Zur Durchführung der Reaktion a) wird das 1,4-Dihydropyridin-derivat mit s-Triazin in einem inerten organischen Lösungsmittel in Gegenwart starker Basen,wie z.B. Alkalialkoholaten oder Natriumhydrid,in einem inerten organischen Lösungsmittel auf Temperaturen von 50-160 °C, vorzugsweise 100-150°C erhitzt. Als Lösungsmittel eignen sich hier vor allem polare Lösungsmittel wie Dimethylsulfoxid, Dimethylformamid, Ethylenglykoldimethylether oder niedere Alkohole wie Ethanol.

Zur Durchführung der Reaktion nach Verfahrensvariante b) wird das entsprechende 1,4-Dihydropyridinderivat mit einer äquivalenten oder überschüssigen Menge Dialkylformamid-dialkylacetal, vorzugsweise in Gegenwart eines aprotischen Lösungsmittels wie Dimethylformamid, Dimethylsulfoxid oder Hexamethylphosphorsäuretriamid, unter Erwärmen umgesetzt. Als geeignete Formamidacetale kommen vor allem Dimethylformamid-dimethylacetal und Dimethylformamid-diethylacetal in Frage.

Das nach der Verfahrensvariante b) erhaltene Zwischenprodukt der allgemeinen Formel XI wird durch Reaktion mit Ammoniak in Gegenwart eines, vorzugsweise protischen Lösungsmittels bei Raumtemperatur oder bei höherer Temperatur, bevorzugt bei der Siedetemperatur des verwendeten Lösungsmittels,in Verbindungen der allgemeinen Formel XIII übergeführt. Als Lösungsmittel sind vor allem niedere Alkohole, wie Methanol oder Ethanol geeignet.

Die Reaktion c) wird vorzugsweise in inerten organischen Lösungsmitteln, insbesondere niederen Alkoholen wie z.B. Methanol, Ethanol oder Isopropanol, durchgeführt. Es ist auch zweckmäßig, bei höheren Temperaturen, vorzugsweise bei der Siedetemperatur des verwendeten Lösungsmittels, zu arbeiten. Die Reaktionsprodukte lassen sich mit bekannten Trennverfahren, wie Kristallisation und/oder Chromatographie isolieren und reinigen.

Die Alkylierung, Amino- und Alkoxyalkylierung der Verbindungen der allgemeinen Formel XIII erfolgt nach allgemein bekannten Methoden, vorzugsweise unter Verwendung eines Halogenwasserstoffakzeptors. Die Reaktion verläuft bei der Wahl geeigneter Reaktionsbedingungen mit hoher Regioselektivität. Die zu erwartenden O-Alkylierungsprodukte werden überraschend nur in geringen Mengen gebildet. Die Trennung bzw. Reinigung der Produkte erfolgt mittels Chromatographie und/oder Kristallisation.

Saure oder basische Verbindungen der allgemeinen Formel I, welche für $R^6$ eine Carboxylgruppe bzw. einen substituierten oder unsubstituierten Aminoalkoxycarbonylrest oder für $R^2$ eine substituierte oder unsubstituierte Aminoalkylgruppe oder/und für $R^5$ eine unsubstituierte oder substituierte Aminogruppe aufweisen, überführt man zum Zwecke der Reinigung und aus galenischen Gründen bevorzugt in kristalline, pharmakologisch verträgliche Salze.

Für den Fall, daß $R^6$ eine Carboxylgruppe darstellt, lassen sich mit Basen wie z.B. Hydroxiden oder Carbonaten entsprechende Salze der Alkali- oder Erdalkalimetalle herstellen. Wenn die Reste $R^2$, $R^5$ und $R^6$ basischen Charakter aufweisen, werden Salze in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Da die erfindungsgemäßen Verbindungen der allgemeinen Formel I an C-4 ein chirales Zentrum aufweisen, können sie entweder als racemische Gemische oder in Form der Enantiomeren vorliegen.

Die Verbindungen der allgemeinen Formel I weisen wertvolle pharmakologische Eigenschaften bei günstiger allgemeiner Verträglichkeit auf.

Aufgrund ihrer gefäßspasmolytischen und thrombozytenaggregationshemmenden Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert, insbesondere weil im Vergleich mit bekannten Präparaten ähnlicher Wirkungsweise negativ inotrope Nebeneffekte weitgehend fehlen. Die 1,6-Naphthyridinon-Derivate der vorliegenden Erfindung sind daher wertvolle Mittel für die Bekämpfung der Herz-Kreislauf-Mortalität, die in Deutschland zur Zeit bei über 50% aller Todesfälle liegt. Ein weiterer Gegenstand der vorliegenden Erfindung ist daher die Verwendung von 1,6-Naphthyridinon-Derivaten der allgemeinen Formel I bei der Bekämpfung von Gefäßerkrankungen.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citrat-Puffer, Ethanol, Komplexbildner (wie Äthylendiamin-tetraessigsäure und deren nichttoxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg; vorzugsweise 20 - 100 mg. Parenteral werden etwa 1 bis 20 mg gegeben.

Die folgenden Beispiele sollen die Erfindung näher erläutern

## Beispiel

**(±)-1,4,5,6-Tetrahydro-2-methyl-4-(3-nitrophenyl)-5-oxo-1,6-naphthyridin-3-carbonsäureethylester**

Variante a)

Eine Suspension von 2,4 g (9 mMol) 3-Nitrobenzylidenacetessigsäureethylester und 1,o g (9 mMol) 2,4-Dihydroxypyridin in 25 ml Ethanol wird mit Ammoniakgas bei Raumtemperatur gesättigt. Unter weiterem Durchleiten von gasförmigem Ammoniak wird 5 Stunden zum Sieden erhitzt. Nach Abziehen des Lösungsmittels im Vakuum wird der Rückstand in 5o ml Chloroform unter Erwärmen gelöst. Die beim Abkühlen ausfallenden Kristalle werden abgesaugt und durch Kristallisation aus Ethanol/Ethylacetat 1 : 1 gereinigt. Man erhält (±)-1,4,5,6-Tetrahydro-2-methyl-4-(3-nitrophenyl)-5-oxo-1,6-naphthyridin-3-carbonsäureethylester als gelbe Nadeln vom Schmp. 285°C (Z).

Variante b)

Zu einer Suspension von 4,95 g (165 mMol) Natriumhydrid (8o %ig in Paraffinöl) in 75 ml trockenem Dimethylformamid wird unter Stickstoffatmosphäre eine Lösung von 56,5 g (15o mMol) 1,4-Dihydro-2,6-dimethyl-4-(3-nitrophenyl)pyridin-3,5-dicarbonsäurediethylester in 25o ml Dimethylformamid getropft. Bei Nachlassen der Gasentwicklung wird kurzzeitig (ca. 1o min) auf 6o °C erwärmt und anschließend werden 12,2 g (15o mMol) s-Triazin in 25o ml Dimethylformamid zugetropft. Die Reaktionsmischung wird 16 Stunden zum Sieden erhitzt, nach dem Abkühlen filtriert und im Vakuum eingeengt. Der dunkle Rückstand wird in 4oo ml n-Hexan aufgekocht. Das ungelöste

Rohprodukt wird nach Abdekantieren des n-Hexans in 5oo ml heißem Ethanol suspendiert und auf eine Säule mit Kieselgel aufgetragen. Anschließend wird mit Methylenchlorid/methanol· Ammoniak (9 : 1 V/V) eluiert und die gewünschte Fraktion zur weiteren Reinigung zweimal aus Ethanol/Ethylacetat (1:1 V/V) umkristallisiert.
Man erhält (±)-1,4,5,6-Tetrahydro-2-methyl-4-(3-nitrophenyl)-5-oxo-1,6-naphthyridin-3-carbonsäureethylester in Form gelber Nadeln vom Schmp. 285 °C (Z).

In analoger Weise, wie in Variante b) beschrieben, erhält man folgende Verbindungen:

0189898

(±)-4-(2-Diethylaminophenyl)-6-ethyl-1,4,5,6-tetrahydro-2-methyl-5-oxo-1,6-naphthyridin-3-carbonsäuremethylester;
Schmp. 214-215°C (aus Ethylacetat);

(±)-2-Amino-1,4,5,6-tetrahydro-4-(2-nitrophenyl)-5-oxo-1,6-naphthyridin-3-carbonsäureethylester;
Schmp. 300°C (Z) (aus Dichlormethan/Ethanol);

(±)-1,4,5,6-Tetrahydro-2-methyl-4-(2-nitrophenyl)-5-oxo-1,6-naphthyridin-3-carbonsäure · Natriumsalz;
Schmp. > 220°C (Z) (aus Ethanol);

(±)-1,4,5,6-Tetrahydro-2-methyl-4-(2-nitrophenyl)-5-oxo-1,6-naphthyridin-3-carbonsäure-(2-methoxyethyl)ester;
Schmp. 265°C (Z) (aus Eisessig);

(±)-1,4,5,6-Tetrahydro-2-methyl-5-oxo-4-(2-trifluormethylphenyl)-1,6-naphthyridin-3-carbonsäureisobutylester;
Schmp. 169-170°C (aus Methanol);

(±)-1,4,5,6-Tetrahydro-2-methyl-5-oxo-4-(2-trifluormethylphenyl)-1,6-naphthyridin-3-carbonsäureisopropylester;
Schmp. 268-270°C (aus Methanol);

(±)-1,4,5,6-Tetrahydro-2-methyl-4-(3-nitrophenyl)-5-oxo-1,6-naphthyridin-3-carbonsäuremethylester;
Schmp. 308-310°C (Z) (aus Methanol);

(±)-1,4,5,6-Tetrahydro-2-methyl-1-(2-morpholinoethyl)-4-(2-nitrophenyl)-5-oxo-1,6-naphthyridin-3-carbonsäuremethylester · Hydrochlorid;
Schmp. 285°C (Z) (aus Methanol/Wasser);

(±)-6-Benzyl-4-(2,3-dichlorphenyl)-1,4,5,6-tetrahydro-2-methyl-5-oxo-1,6-naphthyridin-3-carbonsäuremethylester;
Schmp. 145-148°C (aus Ethylacetat/Methanol);

(±)-4-(2,1,3-Benzoxadiazol-4-yl)-1,4,5,6-tetrahydro-2-methyl-5-oxo-1,6-naphthyridin-3-carbonsäuremethylester;
Schmp. 302-304°C (aus Methanol);

(±)-1,4,5,6-Tetrahydro-2-methyl-4-(3-nitrophenyl)-5-oxo-1,6-naphthyridin-3-carbonsäure-[2-(N-benzyl-N-methylamino)-ethyl]ester;
Schmp. 130-132°C (Z) (aus Ethylacetat);

(±)-1,4,5,6-Tetrahydro-2-methyl-4-(3-nitrophenyl)-5-oxo-1,6-naphthyridin-3-carbonsäure-(2-dimethylaminoethyl)ester;
Schmp. 218-220°C (aus Diisopropylether/Ethanol);

(±)-1,4,5,6-Tetrahydro-2-methyl-5-oxo-4-(2-trifluormethylphenyl)-1,6-naphthyridin-3-carbonsäure-(2-dimethylaminoethyl)-ester
Schmp. 210-212°C (Z) (aus Ethanol);

(±)-1,4,5,6-Tetrahydro-2-methyl-5-oxo-4-(2-trifluormethylphenyl)-1,6-naphthyridin-3-carbonsäure-[2-(N-benzyl-N-methylamino)ethyl]ester • Hydrochlorid
Schmp. 158-160°C (aus Ethylacetat/Ethanol);

(±)-1,4,5,6-Tetrahydro-2-methyl-4-(3-nitrophenyl)-5-oxo-1,6-naphthyridin-3-carbonsäure-(2-methylthioethyl)ester;
Schmp. 233-235°C (aus Ethanol);

(±)-2-Ethyl-1,4,5,6-tetrahydro-8-methyl-5-oxo-4-phenyl-1,6-naphthyridin-3-carbonsäureethylester;
Schmp. 205-207°C (aus Ethylacetat/Ethanol);

(±)-1,4,5,6-Tetrahydro-6-isopropyl-2-methyl-5-oxo-4-(2-trifluormethylphenyl)-1,6-naphthyridin-3-carbonsäureethylester;
ester
Schmp. 211-213°C (aus Diisopropylether/Ethylacetat);

(±)-1,4,5,6-Tetrahydro-2-methyl-5-oxo-4-(2-trifluormethylphenyl)-1,6-naphthyridin -3,8-dicarbonsäurediethylester
Schmp. 267-268°C (aus Ethylacetat)

I. Patentansprüche für die Vertragsstaaten CH, DE, FR, GB, IT

1. 1,6-Naphthyridinon-Derivate der allgemeinen Formel I

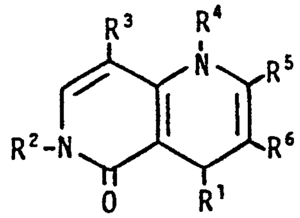

(I)

in welcher

$R^1$ einen unsubstituierten oder substituierten aromatischen oder heteroaromatischen Ring,

$R^2$ Wasserstoff, eine geradkettige oder verzweigte Alkyl-, Alkoxyalkyl-, Arylalkyl- oder eine substituierte oder unsubstituierte Aminoalkylgruppe mit bis zu 10 Kohlenstoffatomen,

$R^3$ Wasserstoff, eine geradkettige oder verzweigte Alkylgruppe oder einen Alkoxycarbonylrest mit bis zu 4 Kohlenstoffatomen,

$R^4$ Wasserstoff oder eine Morpholinoethylgruppe,

$R^5$ eine geradkettige oder verzweigte Alkylgruppe mit bis zu 4 Kohlenstoffatomen oder eine Aminogruppe und

$R^6$ eine Carboxylgruppe oder einen Alkoxycarbonylrest, der bis zu 12 Kohlenstoffatome und gegebenenfalls auch ein Sauerstoff-, Schwefel- oder Stickstoffatom enthält,
bedeuten

sowie gegebenenfalls deren pharmakologisch unbedenkliche Salze.

0189898

2. 1,6-Naphthyridinon-Derivate gemäß Anspruch 1 in welchen

$R^1$ einen Phenylrest, der in 2- oder 3-Stellung durch Halogenatome wie Chlor, Fluor oder Brom, durch Amino-, Diethylamino-, Dimethylamino-, Trifluormethyl- oder Nitro-Gruppen substituiert sein kann oder einen substituierten oder unsubstituierten Benzoxadiazolylrest.

$R^2$ Wasserstoff, einen Ethyl- oder Benzylrest,

$R^3$ Wasserstoff, eine Methyl- oder Ethoxycarbonylgruppe

$R^4$ Wasserstoff oder eine Morpholinoethylgruppe

$R^5$ eine Methyl-, Ethyl- oder Aminogruppe und

$R^6$ eine Carboxylgruppe oder einen Alkoxycarbonylrest der allgemeinen Formel V

$$-CO_2R^{11} \qquad (V)$$

in welcher

$R^{11}$ Wasserstoff, eine Methyl-, Ethyl-, Isopropyl-, Isobutyl-, Methoxyethyl-, Dimethylaminoethyl-, Benzylmethylaminoethyl- oder Methylthioethylgruppe

bedeuten.

3. 1,6-Naphthyridinon-Derivate nach Anspruch 1 und 2 in welchen $R^1$ eine Phenyl-, 3-Nitrophenyl- oder 2-Trifluormethylphenylgruppe, $R^2$ und $R^4$ Wasserstoff bedeuten, und wenn $R^3$ ebenfalls Wasserstoff bedeutet und $R^5$ eine Methylgruppe, dann bedeutet $R^{11}$ eine Dimethylaminoethyl-, Benzylmethylaminoethyl- oder Methylthioethylgruppe, und wenn $R^{11}$ eine Ethylgruppe bedeutet, dann bedeutet $R^3$ eine Methyl- oder Ethoxycarbonylgruppe und $R^5$ eine Methyl- oder Ethylgruppe.

4. 1,6-Naphthyridinon-Derivate gemäß Anspruch 1 und 2 in welchen

$R^1$ eine 2,3-Dichlorphenyl- oder 2-Nitrophenylgruppe,

$R^2$ Wasserstoff- oder eine Benzylgruppe

$R^3$ Wasserstoff,

$R^4$ Wasserstoff- oder eine Morpholinoethylgruppe,

$R^5$ und $R^{11}$ eine Methylgruppe

bedeuten.

5. 1,6- Naphthyridinon-Derivate gemäß Anspruch 1 und 2 in welchen

$R^1$ eine 2-Trifluormethylphenyl-, 2-Nitrophenyl-, 3-Nitrophenyl-, 2-Diethylaminophenyl- oder 2,1,3-Benzoxadiazolylgruppe

$R^2$ Wasserstoff oder eine Ethylgruppe,

$R^3$ und $R^4$ Wasserstoff,

$R^5$ eine Methyl- oder eine Aminogruppe und

$R^{11}$ Wasserstoff, eine Methyl-, Ethyl-, Isopropyl-, Isobutyl- oder Methoxyethylgruppe

bedeuten.

6. Verfahren zur Herstellung von 1,6-Naphthyridinon-Derivaten gemäß der Ansprüche 1 bis 5, dadurch gekennzeichnet, daß man entweder

a) ein Dihydropyridin der allgemeinen Formel IX

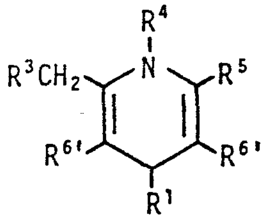

(IX)

in welcher $R^1$, $R^3$, $R^4$ und $R^5$ die oben genannte Bedeutung haben und $R^{6'}$ einen Alkoxycarbonylrest der allgemeinen Formel V darstellt, in Gegenwart einer Base mit s-Triazin umsetzt,

b) ein 1,4-Dihydropyridin der allgemeinen Formel IX mit einem Dialkylformamid-dialkylacetal der allgemeinen Formel X

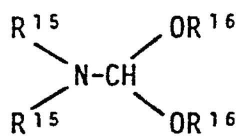

(X)

in welcher die Reste $R^{15}$ gleich oder verschieden sein können und eine Methyl- oder Ethylgruppe bedeuten und die Reste $R^{16}$ jeweils eine Alkylgruppe mit bis zu 4 Kohlenstoffatomen oder gemeinsam eine Alkylengruppe mit bis zu 3 Kohlenstoffatomen bedeuten, zur Reaktion bringt und die erhaltenen Verbindungen der allgemeinen Formel XI

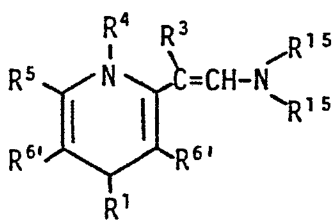

(XI)

in welcher $R^1$, $R^3$, $R^4$, $R^5$, $R^{6'}$ und $R^{15}$ die oben angegebene Bedeutung besitzen, mit Ammoniak umsetzt, oder

c) 2,4-Dihydroxypyridin mit einer Verbindung der allgemeinen Formel XII

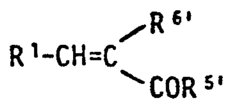

(XII)

in welcher $R^1$ und $R^{6'}$ die oben genannte Bedeutung haben und $R^{5'}$ eine geradkettige oder verzweigte Alkylgruppe mit bis zu 4 Kohlenstoffatomen bedeutet, in Gegenwart von Ammoniak umsetzt.

und die erhaltenen Verbindungen der allgemeinen Formel XIII

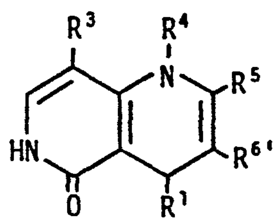

(XIII)

in welcher $R^1$, $R^3$, $R^4$, $R^5$ und $R^{6'}$ die obengenannte Bedeutung haben,

gewünschtenfalls in an sich bekannter Weise mit einer Verbindung der allgemeinen Formel XIV

$X-R^{2'}$ (XIV)

in welcher $R^{2'}$ eine niedere geradkettige oder verzweigte Alkylgruppe, eine Alkoxyalkylgruppe der allgemeinen Formel II oder eine Aminoalkylgruppe der allgemeinen Formel III bedeutet, alkyliert, aminoalkyliert oder alkoxyalkyliert.

7. Verwendung von 1,6-Naphthyridinon-Derivaten nach den Ansprüchen 1 bis 5 bei der Bekämpfung von Gefäßerkrankungen

8. Arzneimittel enthaltend 1,6-Naphthyridinone-Derivate gemäß der Ansprüche 1 bis 6 zur Bekämpfung von Gefäßerkrankungen.

0189898

## II. Patentansprüche für Vertragsstaat AT

1. Verfahren zur Herstellung von 1,6-Naphthyridinon-Derivaten der allgemeinen Formel I

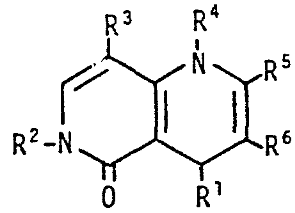

(I)

in welcher

$R^1$ einen unsubstituierten oder substituierten aromatischen oder heteroaromatischen Ring,

$R^2$ Wasserstoff, eine geradkettige oder verzweigte Alkyl-, Alkoxyalkyl-, Arylalkyl- oder eine substituierte oder unsubstituierte Aminoalkylgruppe mit bis zu 10 Kohlenstoffatomen,

$R^3$ Wasserstoff, eine geradkettige oder verzweigte Alkylgruppe oder einen Alkoxycarbonylrest mit bis zu 4 Kohlenstoffatomen,

$R^4$ Wasserstoff oder eine Morpholinoethylgruppe,

$R^5$ eine geradkettige oder verzweigte Alkylgruppe mit bis zu 4 Kohlenstoffatomen oder eine Aminogruppe und

$R^6$ eine Carboxylgruppe oder einen Alkoxycarbonylrest, der bis zu 12 Kohlenstoffatome und gegebenenfalls auch ein Sauerstoff-, Schwefel- oder Stickstoffatom enthält,
bedeuten

sowie gegebenenfalls deren pharmakologisch unbedenkliche Salze, dadurch gekennzeichnet, daß man entweder

a) ein Dihydropyridin der allgemeinen Formel IX

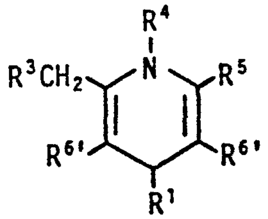

(IX)

in welcher $R^1$, $R^3$, $R^4$ und $R^5$ die oben genannte Bedeutung haben und $R^{6'}$ einen Alkoxycarbonylrest der allgemeinen Formel V darstellt, in Gegenwart einer Base mit s-Triazin umsetzt,

b) ein 1,4-Dihydropyridin der allgemeinen Formel IX mit einem Dialkylformamid-dialkylacetal der allgemeinen Formel X

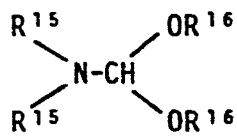

(X)

in welcher die Reste $R^{15}$ gleich oder verschieden sein können und eine Methyl- oder Ethylgruppe bedeuten und die Reste $R^{16}$ jeweils eine Alkylgruppe mit bis zu 4 Kohlenstoffatomen oder gemeinsam eine Alkylengruppe mit bis zu 3 Kohlenstoffatomen bedeuten, zur Reaktion bringt und die erhaltenen Verbindungen der allgemeinen Formel XI

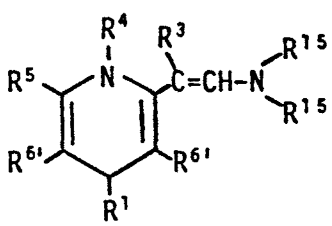

(XI)

in welcher $R^1$, $R^3$, $R^4$, $R^5$, $R^{6'}$ und $R^{15}$ die oben angegebene Bedeutung besitzen, mit Ammoniak umsetzt, oder

c) 2,4-Dihydroxypyridin mit einer Verbindung der allgemeinen Formel XII

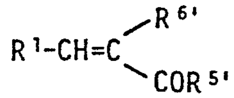

(XII)

in welcher $R^1$ und $R^{6'}$ die oben genannte Bedeutung haben und $R^{5'}$ eine geradkettige oder verzweigte Alkylgruppe mit bis zu 4 Kohlenstoffatomen bedeutet, in Gegenwart von Ammoniak umsetzt.

und die erhaltenen Verbindungen der allgemeinen Formel XIII

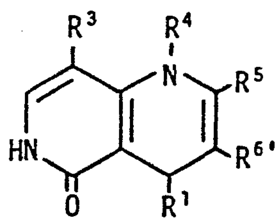

(XIII)

in welcher $R^1$, $R^3$, $R^4$, $R^5$ und $R^{6'}$ die obengenannte Bedeutung haben,

gewünschtenfalls in an sich bekannter Weise mit einer Verbindung der allgemeinen Formel XIV

$$X\text{-}R^{2'} \qquad \text{(XIV)}$$

in welcher $R^{2'}$ eine niedere geradkettige oder verzweigte Alkylgruppe, eine Alkoxyalkylgruppe der allgemeinen Formel II oder eine Aminoalkylgruppe der allgemeinen Formel III bedeutet, alkyliert, aminoalkyliert oder alkoxyalkyliert.

2. Verfahren zur Herstellung von 1,6-Naphthyridinon-Derivaten gemäß Anspruch 1
in welchen

$R^1$ einen Phenylrest, der in 2- oder 3-Stellung durch Halogenatome wie Chlor, Fluor oder Brom, durch Amino-, Diethylamino-, Dimethylamino-, Trifluormethyl- oder Nitro-Gruppen substituiert sein kann oder einen substituierten oder unsubstituierten Benzoxadiazolylrest.

$R^2$ Wasserstoff, einen Ethyl- oder Benzylrest,

$R^3$ Wasserstoff, eine Methyl- oder Ethoxycarbonylgruppe

$R^4$ Wasserstoff oder eine Morpholinoethylgruppe

$R^5$ eine Methyl-, Ethyl- oder Aminogruppe und

$R^6$ eine Carboxylgruppe oder einen Alkoxycarbonylrest der allgemeinen Formel V

$$-CO_2R^{11} \qquad (V)$$

in welcher

$R^{11}$ Wasserstoff, eine Methyl-, Ethyl-, Isopropyl-, Isobutyl-, Methoxyethyl-, Dimethylaminoethyl-, Benzylmethylaminoethyl- oder Methylthioethylgruppe

bedeuten.

3. Verfahren zur Herstellung von 1,6-Naphthyridinon-Derivaten nach Anspruch 1 und 2
in welchen
$R^1$ eine Phenyl-, 3-Nitrophenyl- oder 2-Trifluormethylphenylgruppe, $R^2$ und $R^4$ Wasserstoff bedeuten, und wenn $R^3$ ebenfalls Wasserstoff bedeutet und $R^5$ eine Methylgruppe, dann bedeutet $R^{11}$ eine Dimethylaminoethyl-, Benzylmethylaminoethyl- oder Methylthioethylgruppe, und wenn $R^{11}$ eine Ethylgruppe bedeutet, dann bedeutet $R^3$ eine Methyl- oder Ethoxycarbonylgruppe und $R^5$ eine Methyl- oder Ethylgruppe.

4. Verfahren zur Herstellung von 1,6-Naphthyridinon-Derivaten gemäß Anspruch 1 und 2

in welchen

$R^1$ eine 2,3-Dichlorphenyl- oder 2-Nitrophenylgruppe,

$R^2$ Wasserstoff- oder eine Benzylgruppe

$R^3$ Wasserstoff,

$R^4$ Wasserstoff- oder eine Morpholinoethylgruppe,

$R^5$ und $R^{11}$ eine Methylgruppe

bedeuten.

5. Verfahren zur Herstellung von 1,6-Naphthyridinon-Derivaten gemäß Ansoruch 1 und 2
in welchen

$R^1$ eine 2-Trifluormethylphenyl-, 2-Nitrophenyl-, 3-Nitrophenyl-, 2-Diethylaminophenyl- oder 2,1,3-Benzoxadiazolylgruppe

$R^2$ Wasserstoff oder eine Ethylgruppe,

$R^3$ und $R^4$ Wasserstoff,

$R^5$ eine Methyl- oder eine Aminogruppe und

$R^{11}$ Wasserstoff, eine Methyl-, Ethyl-, Isopropyl-, Isobutyl- oder Methoxyethylgruppe

bedeuten.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0189898 Nummer der Anmeldung

EP 86101103.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP - A2 - 0 133 530 (GÖDECKE AKTIENGESELLSCHAFT) * Patentansprüche 1,3,4 * -- | 1-6,8 | C 07 D 471/04<br>A 61 K 31/44<br>//(C 07 D 471/04)<br>(C 07 D 221:00) |
| A | DE - A1 - 3 027 619 (SANDOZ) * Patentansprüche 1,6-8; Seite 10, Zeilen 5-7 * -- | 1-6,8 | |
| A | US - A - 4 154 837 (HEIDER) * Zusammenfassung * ---- | 1,8 | |

RECHERCHIERTE SACHGEBIETE (Int. Cl.4)

C 07 D 471/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6,8

Unvollständig recherchierte Patentansprüche: -

Nicht recherchierte Patentansprüche: 7

Grund für die Beschränkung der Recherche:

Artikel 52(4) EPÜ; Verfahren zur therapeutischen Behandlung des menschlichen oder tierischen Körpers

| Recherchenort | Abschlußdatum der Recherche | Prüfer |
|---|---|---|
| WIEN | 17-04-1986 | ONDER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 06.82